**Europäisches Patentamt**

**European Patent Office**

**Office européen des brevets**

(19)

(11) Veröffentlichungsnummer: **0 102 544**
**B1**

(12) # EUROPÄISCHE PATENTSCHRIFT

(45) Veröffentlichungstag der Patentschrift:
**01.06.88**

(51) Int. Cl.⁴: **B 01 J 29/28,** B 01 J 37/00,
C 07 C 1/20

(21) Anmeldenummer: **83107786.2**

(22) Anmeldetag: **08.08.83**

(54) Verfahren zur Herstellung von harten, bruchfesten Katalysatoren aus Zeolith-Pulver.

(30) Priorität: **25.08.82 DE 3231498**

(43) Veröffentlichungstag der Anmeldung:
**14.03.84 Patentblatt 84/11**

(45) Bekanntmachung des Hinweises auf die Patenterteilung:
**01.06.88 Patentblatt 88/22**

(84) Benannte Vertragsstaaten:
**BE DE FR GB IT NL**

(56) Entgegenhaltungen:
**EP - A - 0 037 982**
**EP - A - 0 051 741**
**FR - A - 2 130 082**
**US - A - 3 867 279**
**US - A - 4 145 315**

(73) Patentinhaber: **BASF Aktiengesellschaft,
Carl-Bosch-Strasse 38, D-6700 Ludwigshafen (DE)**

(72) Erfinder: **Hoelderich, Wolfgang, Dr., Mannheimer
Strasse 18c, D-6710 Frankenthal (DE)**
Erfinder: **Riekert, Lothar, Dr., Im Eichbaeumle 21,
D-7500 Karlsruhe 1 (DE)**
Erfinder: **Kotter, Michael, Dr., Fruehmessweinberg 22,
D-7320 Bruchsal (DE)**
Erfinder: **Hammon, Ulrich, Rudolfstrasse 27,
D-7500 Karlsruhe 1 (DE)**

**Beschreibung**

Es ist bekannt, Zeolithe verschiedener Art als Katalysatoren oder Katalysatorträger zu verwenden. Synthetische Zeolithe fallen je nach Herstellungsbedingungen in Korngrössen zwischen 0,1 bis 20 µm an. Für ihren Einsatz als Katalysatoren im Festbettreaktor müssen die Primärkristalle durch Verpressen im trocknen oder feuchten Zustand (Tablettieren, Strangpressen) oder durch Aufbaugranulation zu mechanisch stabilen, porösen Formkörpern verarbeitet werden. Die Herstellung und Verarbeitung von porösen Zeolithmassen gelingt nur durch den Zusatz von Hilfsstoffen (Binder, Gleitmittel). Handelt es sich dabei um mineralische Zusätze, die beim Calcinierungsprozess nicht entfernt werden können, so ist deren Einfluss auf die reaktionstechnischen Eigenschaften des geformten Agglomerats zu achten. Das zeolithische Material zeigt bei der Anfeuchtung mit Wasser, sowie bei der normalen Behandlung durch Kneten und Extrudieren ein ungewöhnliches Verhalten. Nach dem Anteigen mit Wasser lässt sich die viskose Masse durch intensives Kneten in einen dünnflüssigen Brei überführen, der durch Feststoffzugabe zunächst eine körnige Konsistenz annimmt, durch anschliessendes Kneten jedoch wieder dünnflüssig wird. Durch Feststoffzusatz kann die Flüssigkeit zwar wiederum engeteigt werden, doch ist damit die Masse keineswegs extrudierfähig. Oberhalb eines kritischen Wasser/Feststoffverhältnisses verflüssigt sich die Masse im Extruder bzw. beim Durchtritt durch die Düsenplatte. Unterhalb dieses verhältnisses hingegen verfestigt sie sich im Bereich der Düsenplatte derart, dass ein weiterer Transport unmöglich wird. Es stellte sich daher die Aufgabe, mechanisch stabile Formkörper aus Zeolith-Pulver herzustellen, die keine Tonmineralien oder $Al_2O_3$ als Binder enthalten und wobei die obengenannten Nachteile bei der Verarbeitung nicht auftreten.

Es wurde nun gefunden, dass man harte, bruchfeste Zeolithkatalysatoren der Pentasilfamilie erhält, wenn man das Zeolithpulver mit Wasser und viskositätssteigernden organischen Zusatzstoffen und einem Kieselsäureester versetzt, die Masse verformt, trocknet und calciniert.

Weiterhin zeigen die erfindungsgemässen Zeolithkatalysatoren einen selektivitätssteigernden Einfluss auf die Methanol- bzw. Dimethyletherumwandlung zu niederen Olefinen. Auch wirkt sich die in der Erfindung beschriebene Konfektionierungsmethode positiv auf das Standzeitverhalten des Katalysators aus.

Die Verformung der Katalysatormasse kann durch Versprühen in einem Sprühturm oder aber durch Kneten und anschliessendes Strangpressen vorgenommen werden. Im letzteren Falle kann die Festigkeit der Strangpresslinge erhöht werden, wenn man diese nach dem Trocknen mit einer Lösung von 2,5% Tetramethylorthosilikat in Tetrachlorkohlenstoff tränkt.

Eine spezielle Ausführungsform des Verfahrens besteht z.B. darin, dass Zeolithpulver in einem Kneter einem Gemisch aus Wasser und Hydroxyethylcellulose zuzusetzen, bis sich ein hochviskoder Teig gebildet hat und anschliessend unter Kneten zu der so gebildeten extrudierbaren Masse einen Kieselsäureester zuzumischen, die Masse auszupressen, die Strangpresslinge zu trocknen und anschliessend zu calzinieren.

Die Verformung durch Versprühen der Masse bietet die vorteilhafte Möglichkeit die für Wirbelschichtkatalysatoren gewünschte Korngrösse in laufender Weise einzustellen.

Die weitere Behandlung der Strangpresslinge erfolgt durch Trocknung und Calzinierung. Die Trocknung erfolgt im allgemeinen bei Temperaturen von 30 bis 140 °C. Die Calzinierung wird bei 400 bis 800 °C vorgenommen.

Besonders hohe Festigkeit des Katalysators erzielt man, wenn die Trocknung mit hohem Sättigungsgrad an Wasser im Trocknungsmedium durchgeführt wird.

Anhand eines einfachen Modells lässt sich das ungewöhnliche rheologische Verhalten des feuchten Zeolith-Pulvers deuten: Beim Anfeuchten des Pulvers bilden sich zunächst feuchte Agglomerate, die durch Flüssigkeitsbrücken zusammengehalten werden. Ihre gegenseitige Verschiebbarkeit wird durch Formschluss verhindert.

Das gleiche Ergebnis erhält man bei Zugabe von Feststoff zur Flüssigkeit. Hier werden zwar die Primärteilchen zunächst dispergiert, bei hinreichender Feststoffzugabe wird es jedoch immer dann zur Bildung von Agglomeraten (Klumpen) kommen, wenn die Mischung von Feststoff und Flüssigkeit nicht über das gesamte Volumen des Kneters gleichmässig erfolgt. Lange Verweilzeiten im Kneter bewirken eine Auflösung der Agglomerate durch die ständig wirksamen Scherkräfte im Probenraum. Die Primärteilchen werden mit einem Flüssigkeitsfilm umgeben und damit gegeneinander verschiebbar; die Masse wird «dünnflüssig».

Da das Extrudat im Extruder sowie beim Durchtritt durch die Düse ebenfalls Scherkräften ausgesetzt wird, kann es bei hinreichendem Flüssigkeitsgehalt zu einer Erniedrigung der Viskosität der Masse kommen. Bei Unterschreiten eines kritischen Flüssigkeit/Feststoffverhältnisses hingegen werden die Primärteilchen oder auch Agglomerate durch Druck im Förderraum und insbesondere vor der Düsenplatte so zusammengedrückt, dass bei direktem Kontakt der Feststoffteilchen durch Haft- und Formschluss deren Verschiebbarkeit aufgehoben wird. Durch Zusatz einer makromolekularen Substanz lässt sich die Bildung von Agglomeraten verhindern, eine Haftwirkung bei begrenzter Verschiebbarkeit der Primärteilchen gegeneinander bewirken und ein für den Extruder/Pressvorgang optimaler Flüssigkeitsgehalt (Zähigkeit) einstellen. Die Primärteilchen werden von Makromoleküle enthaltenden stabilen Flüssigkeitsbrücken zusammengehalten, ohne dass ihre gegenseitige Verschiebbarkeit völlig aufgehoben würde. Unter Druckeinwirkung ist ein derartiges Gebilde in Grenzen stabil.

Die Festigkeit der unter Zusatz von Hydroxyethylcellulose (HEC) hergestellten, getrockneten Presslinge ist zwar beachtlich (bei 2% HEC 300 N), jedoch nimmt diese bei Calcinieren und Entfernen des als Bindemittel wirksamen Zusatzes drastisch ab. Werden jedoch in der porösen Struktur durch Hydrolyse bzw. thermische Zersetzung eines Kieselsäureesters Feststoffbrücken aus $SiO_2$ gebildet, so lassen sich beachtliche Druckfestigkeiten in der calzinierten Form erzielen. Dabei ist anzunehmen, dass die Primärteilchen zunächst durch Flüssigkeitsbrücken miteinander verbunden sind, die beim Trocknungsvorgang durch Feststoffbrücken ersetzt werden. Offensichtlich spielt es dabei nur eine untergeordnete Rolle, ob der hier verwendete Brückenbildner direkt der feuchten Masse zugesetzt wird, oder ob die Zugabe in einem zweiten Verfahrensschritt erfolgt. Für die Festigkeit der resultierenden Stränge scheint es unerheblich, ob die zugesetzte organische Siliciumverbindung bereits während des Knetens und Extrudierens oder später hydrolysiert wird.

Die erfindungsgemäss hergestellten Zeolithkatalysatoren zeichnen sich durch besondere Härte und Bruchfestigkeit aus. Sie sind deshalb besser als Wirbelkatalysatoren für die Umsetzung von Methanol und/oder Dimethylether zu Kohlenwasserstoffen, insbesondere zu $C_2$–$C_4$-Olefinen geeignet.

Die katalytische Aktivität und die Selektivität der Bildung niederen Olefinen aus Dimethylether (Methanol) werden durch das in den Agglomeraten gebildete $SiO_2$ nicht nachteilig beeinflusst.

Das Sorptionsvermögen des Zeolith-Materials wird bei diesem Verfahren der Bildung mechanisch stabiler Formkörper nicht vermindert (vgl. letzte Spalte Tabelle). Molekularsiebe (Zeolithe) werden in der Technik vornehmlich zur Stofftrennung durch Absorption (z.B. Trocknung von Gasen) eingesetzt. Auch in diesen Anwendungsfällen dürfte es vorteilhaft sein, nach dem hier beschriebenen Verfahren mechanisch widerstandsfähige makroporöse Agglomerate herzustellen, die weniger als 5 Gew.-% eines inerten Bindemittels enthalten.

Als Ausgangsmaterial für die im folgenden beschriebenen Versuche diente ein feinkristalines Zeolith-Pulver, das nach den Ergebnissen eines Röntgenbeugungsexperimentes in die Pentasilfamilie einzuordnen ist.

Beispiel 1

In 2000 g 25%ige Hexamethylendiaminlösung wird bei 60 °C 160 g Aerosil und frisch gefälltes $Al(OH)_3$ eigetragen. Das $Al(OH)_3$ wird aus 61,4 g $Al(NO_3)_3 \cdot 9 H_2O$ durch Fällen mit Ammoniak hergestellt. Das $SiO_2/Al_2O_3$-Molverhältnis in der Mischung beträgt 32,6. Die Mischung wird dann so lange gerührt bis sie homogen ist und anschliessend in einem Stahlautoklaven 5 Tage auf 150 °C unter ihrem Eigendruck erhitzt. Das kristalline Produkt wird filtriert, gewaschen und bei 100 °C/ 16 h getrocknet und bei 500 °C/16 h calciniert. Laut Röntgenanalyse besteht es aus gut kristallisiertem Aluminiumzeolith.

Beispiel 2

Im Kneter werden Wasser und 2% Hydroxyethylcellulose (HEC) zu einer zähen Flüssigkeit gemischt, der nach und nach soviel Zeolith-Pulver zugesetzt wird, bis sich ein hochviskoser Teig gebildet hat. Diese Masse lässt sich problemlos zu Strängen extrudieren. Nach Trocknen und Entfernen der Cellulose durch Calcinieren bei 500 °C für zwei Stunden an Luft erhält man Formkörper mit hoher Makroporosität. Die Druckfestigkeit von 4 mm-Strängen bei 10 mm Länge beträgt bei Belastung der Mantelfläche 80 N. Die Eigenschaften der erhaltenen Strangpresslinge sind in der Tabelle wiedergegeben.

Beispiel 3

Der extrudierbaren Masse, deren Herstellung in Beispiel 2 beschrieben ist, wird ein Kieselsäureester (hier Tetramethylorthosilikat) unter Kneten zugemischt. Die weitere Behandlung der Masse erfolgt wie im Beispiel 2 beschrieben. Man erhält danach mechanisch stabile Formkörper, deren Druckfestigkeit bei einem Zusatz von 5% Brückenbildner den beachtlichen Wert von 340 N erreicht (vgl. Tabelle 1).

Tabelle 1: Eigenschaften verschieden hergestellter Strangpreßlinge

| Verfahren | Gew.-Anteil $SiO_2$ (aus Tetramethylsilikat) % | Festigkeit (N) | Makroporosität (Vol%) | Reaktion von Dimethylether bei 600 K und $p_{DMÄ} = 39$ mbar im Differentialreaktor | | Sorption von n-Hexan bei 293 K $P_{C_6} = 0,5$ mbar (Gew.%) |
|---|---|---|---|---|---|---|
| | | | | Reaktionsgeschwindigkeit in mol $g^{-1}s^{-1}$ | Gew.% $(C_2H_4 + C_3H_6)$ in entstandenen Kohlenwasserstoffen | |
| (1) | – | 90 | 50 | $1,2 \cdot 10^{-5}$ | 31 | 8,1 |
| (2) | 5 | 340 | 50 | | | |
| (2) | 2,2 | 203 | 50 | $1,4 \cdot 10^{-5}$ | 32 | 7,9 |
| (3) | 2,5 | 270 | 50 | | | |
| (3) | 1,5 | 200 | 50 | $1,6 \cdot 10^{-5}$ | 31 | 8,0 |

**Beispiel 4**

Strangpresslinge, die nach dem in Beispiel 2 beschriebenen Verfahren hergestellt wurden, werden anschliessend mit einer Lösung von 2,5% Tetramethylorthosilikat in Tetrachlorkohlenstoff imprägniert, getrocknet und bei 500 °C für 2 Stunden calziniert. Die Festigkeit ist durch den zusätzlichen Verfahrensschritt mehr als verdreifacht worden (s. Tabelle 1).

**Beispiel 5**

Die Pentasil-Presslinge, hergestellt nach Verfahren 2, 3 und 4 wurden in einem Rohrreaktor hinsichtlich ihres reaktionstechnischen Verhaltens bei der Umsetzung von Dimethylether im Temperaturbereich von 580 bis 625 K bei einem Partialdruck des Dimethylethers von 39 mbar untersucht. Bei einer Kontaktbelastung von 0,15 mol DMÄ $g^{-1}h^{-1}$ lag der Umsatz zwischen 20 und 40% (Daten s. Tabelle 1).

**Beispiele 6 und 7**

Der in Beispiel 3 hergestellte Zeolithkatalysator (A) wird mit einem Katalysator (B) verglichen, der aus Zeolithpulver (Beispiel 1) und Boehmit im Verhältnis 60:40 in üblicher Weise konfrontiert wurde. Als Testreaktion wurde die Methanolumwandlung zu niederen Olefinen gewählt. Der Vergleich der Testergebnisse (Tabelle 2) zeigt, dass die Ausbeute an Olefin im $C_2–C_4$-Bereich durch die erfindungsgemässe Konfektionierung erhöht wird. Das Methanol wird quantitativ umgesetzt. Die angegebenen Ausbeuten sind auf eingesetztes $CH_2$ bezogen.

**Tabelle 2**

| Beispiel | 6 | 7 |
|---|---|---|
| Katalysator | A | B |
| Temperatur | 450°C | |
| WHSV | $2,74^{-1}$ | |
| $CH_3OH$ | 25% | |
| $CH_4$ % | 1,5 | 1,5 |
| $C_2H_4$ % | 7,8 | 6,8 |
| $C_2H_6$ % | / | / |
| $C_3H_6$ % | 35,7 | 30,9 |
| $C_3H_8$ % | 1,2 | 1,3 |
| $C_4$ % | 18,1 | 15,6 |
| $C_5^+$ % | 34,6 | 38,3 |
| $^+$Laufzeit h | 51 | 29 |
| g $CH_3OH$/g Kontakt | 138 | 78 |

$^+$Laufzeit bis zur ersten Regenerierung des Kontaktes.

Die Laufzeit des Zeolithkatalysators, bezogen auf eingesetztes Katalysatorvolumen wird durch die erfindungsgemässe Massnahme gesteigert.

**Beispiel 8**

Es wird die Herstellung eines sprühgetrockneten Zeolithkatalysators beschrieben, der sich als Wirbelkatalysator einsetzen lässt.

Das in Beispiel 1 hergestellte Zeolithpulver wird mit Wasser, 2 Gew.-% Hydroxyethylcellulose (HEC) und 2,5 Gew.-% Tetramethylorthosilikat zu einer pumpbaren Suspension angemaischt. Der Feststoffgehalt dieser Maische entspricht ca. 30 Gew.-%. Die Suspension wird in einem Sprühtrockner zu einem Zeolithkatalysator mit einer Teilchengrösse von 50 bis 200 μm versprüht.

**Patentansprüche**

1. Verfahren zur Herstellung von harten, bruchfesten Zeolithkatalysatoren der Pentasilfamilie, dadurch gekennzeichnet, dass man das Zeolithpulver mit Wasser und viskositätssteigernden organischen Zusatzstoffen und einem Kieselsäureester versetzt, die Masse verformt, trocknet und calziniert.

2. Verfahren zur Herstellung von Zeolithkatalysatoren nach Anspruch 1, dadurch gekennzeichnet, dass man die Verformung durch Versprühen der Masse in einem Sprühturm vornimmt.

3. Verfahren zur Herstellung von Zeolithkatalysatoren nach Anspruch 1, dadurch gekennzeichnet, dass man die Verformung durch Kneten der Masse und anschliessendes Strangpressen vornimmt.

4. Verfahren nach Anspruch 3, dadurch gekennzeichnet, dass man die Strangpresslinge nach dem Trocknen mit einer Lösung von 2,5% Tetramethylorthosilikat in Tetrachlorkohlenstoff tränkt.

**Claims**

1. A process for the preparation of a hard, fracture-resistant zeolite catalyst of the pentasil family, wherein water, an organic additive which increases the viscosity, and a silicate are added to the zeolite powder, and the material is molded, dried and calcined.

2. A process for the preparation of a zeolithe catalyst as claimed in claim 1, wherein molding is carried out by spraying the material in a spray tower.

3. A process for the preparation of a zeolite catalyst as claimed in claim 1, wherein molding is carried out by kneading the material and then extruding it.

4. A process as claimed in claim 3, wherein the extrudates are dried and then impregnated with a solution of 2.5% of tetramethyl orthosilicate in carbon tetrachloride.

**Revendications**

1. Procédé de préparation de catalyseurs durs, résistant à l'écrasement, à base de zéolithes de la famille du pentasil, caractérisé en ce que l'on mélange de la zéolithe pulvérulente avec de l'eau, des additifs organiques augmentant la viscosité et un ester de l'acide silicique, on façonne la masse obtenue, puis on la sèche et on la calcine.

2. Procédé de préparation de catalyseurs à base de zéolithes suivant la revendication 1, caractérisé en ce que le façonnage est réalisé par la pulvérisation de la masse dans une colonne de pulvérisation.

3. Procédé de préparation de catalyseurs à base de zéolithes suivant la revendication 1, caractérisé en ce que le façonnage est réalisé par malaxage de la masse, suivi de son extrusion en boudins.

4. Procédé suivant la revendication 3, caractérisé en ce que les boudins extrutés sont séchés, puis imprégnés d'une solution à 2,5% d'ortho-silicate tétraméthylique dans le tétrachlorure de carbone.